# EUROPÄISCHE PATENTSCHRIFT

(11) **EP 0 463 187 B1**
(45) Veröffentlichungstag und Bekanntmachung des Hinweises auf die Patenterteilung: **03.05.1995**
(21) Anmeldenummer: 90111569.1
(22) Anmeldetag: 19.06.1990
(51) Int. Cl.: A61B 6/08, A61B 6/00

(54) **Medizinische Vorrichtung**
Medical device
Appareil à usage médical

(43) Veröffentlichungstag der Anmeldung: 02.01.1992
(73) Patentinhaber: SIEMENS AKTIENGESELLSCHAFT, 80333 München (DE)
(72) Erfinder: Niepel, Günter, D-8520 Erlangen (DE)

(56) Entgegenhaltungen:
- EP-A- 0 365 737
- DE-A- 3 119 295
- DE-B- 1 014 285
- DE-B- 1 028 202
- DE-U- 8 612 533
- US-A- 4 298 801
- Siemens-Prospekt "Angiostar"( Bestellt-Nr. A91003-M1031-G589-01-7600)

## Beschreibung

Aus dem Siemens Prospekt Angiostar (Bestell-Nr. A91003-M1031-G589-01-7600) ist ein Angiographie-Arbeitsplatz mit einem Röntgenstrahler auf einem darauf ausgerichteten Strahlenempfänger und mit einer Patientenlagerungsvorrichtung bekannt, die mittels an einem Bedienkästchen vorgesehenen Joysticks verstellbar sind. Eine ebenfalls vorgesehene Anzeigevorrichtung zeigt die räumliche Ausrichtung der zentralen Achse des Röntgenstrahlers sowie ein Untersuchungsobjekt.

Aus der DE-A-31 19 295 ist eine medizinische Vorrichtung mit einem Ultraschall-Array als Strahlensender bekannt, bei dem nicht nur der Abstand des Fokus von der Abstrahlfläche des Array, sondern zusätzlich auch noch zur Durchführung von Strahlschwenks der Winkel der Verbindungsgraden zum Fokus verändert werden kann.

Es sind weitere medizinische Vorrichtungen, beispielsweise zur Strahlen- bzw. Stoßwellentherapie bekannt, wobei die räumliche Ausrichtung der zentralen Achse eines Strahlensenders durch Bedienelemente eingestellt werden kann.

Aufgabe der Erfindung ist es, eine medizinische Vorrichtung der eingangs genannten Art so auszuführen, daß die Bedienung zur räumlichen Ausrichtung der zentralen Achse des Strahlensenders vereinfacht ist.

Diese Aufgabe wird erfindungsgemäß durch die Merkmale des Anspruches 1 gelöst.

Vorteil der Erfindung ist, daß somit ein schnelles und bequemes Verstellen des Strahlsenders möglich ist. Besitzt das Bedienelement eine Markierung, die mit der räumlichen Ausrichtung der zentralen Achse korreliert, so wird bei einer Verstellung des Bedienelementes durch die Markierung die räumliche Ausrichtung der zentralen Achse vorteilhaft angezeigt.

Vorteilhaft ist, wenn das Bedienelement stabförmig ausgebildet ist und wenn die räumliche Ausrichtung der zentralen Achse mit der räumlichen Ausrichtung der Längsachse des Bedienelementes korreliert. Die zentrale Achse des Strahlensenders kann somit durch einfache Weise, nämlich der räumlichen Ausrichtung des Bedienelementes, beispielsweise in bezug zu einem Untersuchungsobjekt, eingestellt werden.

Vorteilhaft ist, wenn eine Verstellung der räumlichen Ausrichtung der Achse des Bedienelementes eine Verstellung des Strahlensenders derart bewirkt, daß die räumliche Ausrichtung der zentralen Achse der Verstellung der Achse des Bedienelementes nachgeführt wird. Durch die Verstellung des Bedienelementes und somit der Achse des Bedienelementes kann die räumliche Ausrichtung der zentralen Achse des Strahlensenders vorbestimmt werden.

Ein besonders schnelles und exaktes räumliches Ausrichten des Strahlensenders ist ermöglicht, wenn die Geschwindigkeit der Nachführung von der Differenz der räumlichen Ausrichtung der Achse des Bedienelementes zur räumlichen Ist-Ausrichtung der zentralen Achse abhängt, wobei bei einer großen Differenz eine Nachführung mit hoher Geschwindigkeit und wobei bei einer kleinen Differenz eine Nachführung mit geringer Geschwindigkeit erfolgt.

Vorteilhaft ist, wenn eine der räumlichen Ausrichtung der Achse des Bedienelementes entsprechende räumliche Ausrichtung der zentralen Achse erst dann erfolgt, wenn ein Schaltelement betätigt wird. Hierdurch erfolgt die Ausrichtung des Strahlensenders erst dann, wenn die räumliche Ausrichtung der zentralen Achse durch das Ausrichten der Achse des Bedienelementes vorbestimmt ist. Der Verschleiß der Lager und Verstellmittel ist somit reduziert. Außerdem ist einem unbeabsichtigten Verstellen der Anordnung entgegen gewirkt.

Da sich die räumliche Ausrichtung des Strahlensenders und damit die der zentralen Achse bei bestimmten Behandlungen oder Untersuchungen wiederholt, ist es vorteilhaft, wenn eine zweite Steuervorrichtung zum Abrufen und Bewirken mindestens einer vorbestimmten Ausrichtung der zentralen Achse vorgesehen ist. Die Ausrichtung des Strahlensenders kann somit auf schnelle und einfache Weise erfolgen. In Verbindung hiermit ist es vorteilhaft, wenn für das Bedienelement Verstellmittel vorgesehen sind, die bei einer Aktivierung der zweiten Steuervorrichtung das räumliche Ausrichten der Achse des Bedienelementes entsprechend der räumlichen Ausrichtung der zentralen Achse bewirken.

Vorteilhaft ist es, wenn das Bedienelement nur solche Ausrichtungen einnehmen kann, die auch vom Strahlensender eingenommen werden können.

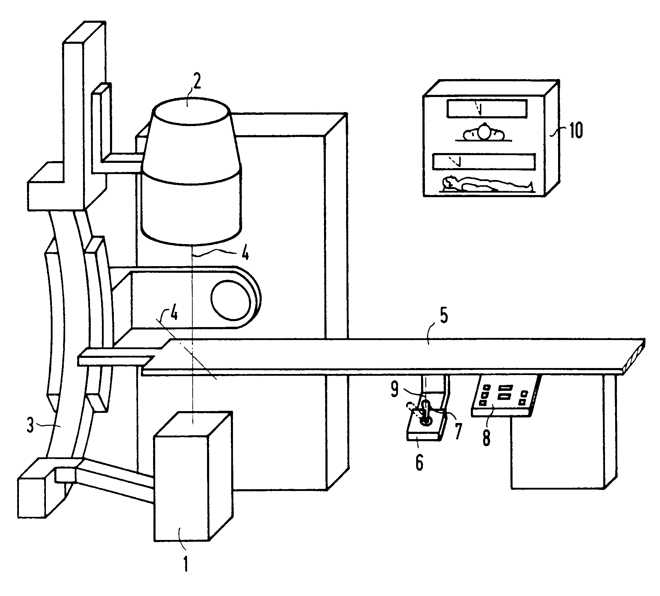

Weitere Vorteile und Einzelheiten der Erfindung ergeben sich aus der nachfolgenden Beschreibung eines Ausführungsbeispieles anhand der Zeichnung.

Gezeigt ist eine medizinische Vorrichtung mit einem erfindungswesentlichen Strahlensender 1 und einem Strahlenempfänger 2, die an den Enden eines verstellbar gelagerten C-Bogens 3 gelagert und aufeinander derart ausgerichtet sind, daß die zentrale Achse 4 des Strahlensenders 1 auf den Zentralbereich des Strahlenempfängers 2 auftrifft. Die zentrale Achse 4 repräsentiert den Zentralstrahl des Strahlensenders 1. An einer Lagerung 5 für ein Untersuchungsobjekt ist eine erste Steuervorrichtung 6, auf das ein Bedienelement 7 wirkt, und eine zweite Steuervorrichtung 8 gehalten. Das Bedienelement 7 besitzt eine Achse 9, die der Längsachse des Bedienelementes 7 entspricht, wenn dieses vorzugsweise stabförmig ausgebildet ist. Eine Verstellung des Bedienelementes 7 wirkt auf die erste Steuervorrichtung 6, die in Verbindung mit weiteren Steuerelementen das räumliche Ausrichten der zentralen Achse 4 entsprechend der räumlichen Ausrichtung der Achse 9 des Bedienelementes 7 bewirkt. Wird das Bedienelement 7 beispielsweise aus der gezeigten senkrechten Ausrichtung in die strichpunktierte Ausrichtung verstellt, so kann bei Bedienung eines Schaltelementes, das beispielsweise am Bedienelement 7 vorgesehen ist, ein Steuersignal erfolgen, durch das eine Verstellung der zentralen Achse 4 in die strichpunktiert gezeichnete Position bewirkt wird. Auch hier entspricht die räumliche Ausrichtung der strichpunktiert gezeichneten zentralen Achse 4 der des strichpunktiert gezeichneten Bedienelementes 7. Vorteilhafterweise kann das Bedienelement 7 nur solche räumliche Ausrichtungen einnehmen, die auch vom Strahlensender 1 und gegebenenfalls vom Strahlenempfänger 2 eingenommen werden können. Bei voreingestellter Ausrichtung des Bedienelementes 7 kann die Nachführung der zentralen Achse 4 derart erfolgen, daß bei einer großen Differenz der räumlichen Ausrichtung der Achse 9 des Bedienelementes 7 zur räumlichen Ist-Ausrichtung der zentralen Achse 4 eine Verstellung mit einer hohen Geschwindigkeit und bei einer kleinen Differenz mit geringer Geschwindigkeit erfolgt.

Mittels der zweiten Steuervorrichtung 8 können beispielsweise vorbestimmte Ausrichtungen der zentralen Achse 4 abgerufen und bewirkt werden, die bei bestimmten Behandlungen oder bei Standarduntersuchungen häufig Anwendung finden. Das Steuersignal der zweiten Steuervorrichtung 8 kann beispielsweise auch auf Verstellmittel für das Bedienelement 7 wirken, so daß die Ausrichtung des Bedienelementes 7 entsprechend der bei Betätigung eines Schaltmittels der zweiten Steuervorrichtung 8 ausgewählten Ausrichtung der zentralen Achse 4 erfolgt. Als Verstellmittel für das Bedienelement 7 können beispielsweise Schrittmotore eingesetzt werden, die dann in der ersten Steuervorrichtung 6 angeordnet sind.

Gezeigt ist auch eine Anzeigevorrichtung 10, die die räumliche Ausrichtung der zentralen Achse 4 und/oder die räumliche Ausrichtung der Achse 9 des Bedienelementes 7 in bezug zu einem bildlich dargestellten Untersuchungsobjekt anzeigt. In gestrichelter Linie kann so beispielsweise die Ausrichtung der Achse 9 des Bedienelementes 7 und damit die gewünschte Ausrichtung der zentralen Achse 4 und durch eine Voll-Linie, beispielsweise die Ist-Ausrichtung der zentralen Achse 4, angezeigt werden. Selbstverständlich ist es auch möglich, die räumliche Ausrichtung der zentralen Achse 4 sowie die räumliche Ausrichtung der Achse 9 des Bedienelementes 7 durch Zahlen anzugeben.

Besitzt die erste Steuervorrichtung 6 eine Kugeloberfläche entlang der das Bedienelement 7 verstellbar ist, so ist der Verstellbereich des Bedienelementes 7 erweitert.

Selbstverständlich kann das Bedienelement 7 nicht nur stabförmig, sondern auch kugelförmig ausgebildet sein. Eine Verstellung des kugelförmigen Bedienelementes um seinen zentralen Punkt bewirkt auch hierbei eine Verstellung des Strahlensenders 1 und gegebenenfalls des Strahlenempfängers 2. Vorteilhaft besitzt das kugelförmige Bedienelement eine Markierung, die auch als Ausnehmung ausgeführt sein kann, wobei dann die zentrale Achse 4 des Strahlensenders 1 mit einer, den Mittelpunkt der Markierung und den zentralen Punkt des kugelförmigen Bedienelementes verbindenden Achse korreliert. Die Markierung zeigt somit die räumliche Ausrichtung der zentralen Achse 4 an.

Die Erfindung ist nicht auf die gezeigte medizinische Vorrichtung beschränkt, sie kann beispielsweise auch bei einem Lithotripsie-Arbeitsplatz und einem Strahlentherapiegerät Anwendung finden.

## Patentansprüche

1. Medizinische Vorrichtung mit einem eine zentrale Achse (4) aufweisenden Strahlensender (1), der einen Zentralstrahl entlang der zentralen Achse (4) aussendet, wobei die zentrale Achse (4) räumlich ausrichtbar ist und mit einem Bedienelement (7) mit einer räumlich ausrichtbaren Bedienelementachse (9) das auf eine Steuervorrichtung (6) zum Verändern der räumlichen Ausrichtung der zentralen Achse (4) wirkt, **dadurch gekennzeichnet,**
daß das Bedienelement (7) derart auf die Steuervorrichtung (6) wirkt, daß die räumliche Ausrichtung der zentralen Achse (4) des Strahlensenders (1) mit der räumlichen Ausrichtung der Bedienelementachse (9) korreliert.

2. Medizinische Vorrichtung nach Anspruch 1, wobei das Bedienelement (7) stabförmig ausgebildet ist und wobei die räumliche Ausrichtung der zentralen Achse (4) mit der räumlichen Ausrichtung der Längsachse (9) des Bedienelementes (7) korreliert.

3. Medizinische Vorrichtung nach Anspruch 1 oder 2, die einen auf den Strahlensender (1) ausgerichteten Strahlenempfänger (2) aufweist, und wobei die zentrale Achse (4) des Strahlensenders (1) auf den Zentralbereich des Strahlenempfängers (2) auftrifft.

4. Medizinische Vorrichtung nach einem der Ansprüche 1 bis 3, wobei eine Verstellung der räumlichen Ausrichtung der Achse (9) des Bedienelementes (7) eine Verstellung des Strahlensenders (1) derart bewirkt, daß die räumliche Ausrichtung der zentralen Achse (4) der Verstellung der Achse (9) des Bedienelementes (7) nachgeführt wird.

5. Medizinische Vorrichtung nach Anspruch 4, wobei die Geschwindigkeit der Nachführung von der Differenz der räumlichen Ausrichtung der Achse (9) des Bedienelementes (7) zur räumlichen Ist-Ausrichtung der zentralen Achse (4) abhängt, wobei bei einer großen Differenz eine Nachführung mit hoher Geschwindigkeit und wobei bei einer kleinen Differenz eine Nachführung mit geringer Geschwindigkeit erfolgt.

6. Medizinische Vorrichtung nach Anspruch 4 bis 5, wobei eine der räumlichen Ausrichtung der Achse (9) des Bedienelementes (7) entsprechende räumliche Ausrichtung der zentralen Achse (4) erst dann erfolgt, wenn ein Schaltelement betätigt wird.

7. Medizinische Vorrichtung nach einem der Ansprüche 1 bis 6 mit einer zweiten Steuervorrichtung (8) zum Abrufen und Bewirken mindestens einer vorbestimmten Ausrichtung der zentralen Achse (4).

8. Medizinische Vorrichtung nach Anspruch 7, wobei für das Bedienelement (7) Verstellmittel vorgesehen sind, die bei einer Aktivierung der zweiten Steuervorrichtung (8) das räumliche Ausrichten der Achse (9) des Bedienelementes (7) entsprechend der räumlichen Ausrichtung der zentralen Achse (4) bewirken.

9. Medizinische Vorrichtung nach einem der Ansprüche 1 bis 8, wobei eine Anzeigevorrichtung (10) für die räumliche Ausrichtung der zentralen Achse (4) sowie für die räumliche Ausrichtung der Achse (9) in bezug zu einem bildlich dargestellten Untersuchungsobjekt vorhanden ist.

10. Medizinische Vorrichtung nach einem der Ansprüche 1 bis 9, wobei das Bedienelement (7) nur solche Ausrichtungen einnehmen kann, die auch vom Strahlensender (1) eingenommen werden können.

## Claims

1. Medical apparatus having a radiation transmitter (1) with a central axis (4), which transmits a central beam along the central axis (4), the spatial orientation of the central axis (4) being adjustable, and having an operating element (7) with an operating element axis (9), which has an adjustable spatial orientation and which acts on a control device (6) for changing the spatial orientation of the central axis (4), characterised in that the operating element (7) acts on the control device (6) in such a manner that the spatial orientation of the central axis (4) of the radiation transmitter (1) correlates with the spatial orientation of the operating element axis (9).

2. Medical apparatus according to claim 1, wherein the operating element (7) is rod-shaped and wherein the spatial orientation of the central axis (4) correlates with the spatial orientation of the longitudinal axis (9) of the operating element (7).

3. Medical apparatus according to claim 1 or 2 having a radiation receiver (2) aligned with the radiation transmitter (1), wherein the central axis (4) of the radiation transmitter (1) is aimed at the central region of the radiation receiver (2).

4. Medical apparatus according to one of claims 1 to 3, wherein displacement of the spatial orientation of the axis (9) of the operating element (7) effects displacement of the radiation transmitter (1) in such a manner that the spatial orientation of the central axis (4) follows the displacement of the axis (9) of the operating element (7).

5. Medical apparatus according to claim 4, wherein the speed of follow-up depends on the differential between the spatial orientation of the axis (9) of the operating element (7) and the actual spatial orientation of the central axis (4), wherein in the case of a large differential follow-up is effected at a high speed, and in the case of a small differential follow-up is effected at a low speed.

6. Medical apparatus according to claim 4 to 5, wherein the spatial orientation of the central axis (4) is only adjusted according to the spatial orientation of the axis (9) of the operating element (7) if a switching element is actuated.

7. Medical apparatus according to one of claims 1 to 6, having a second control device (8) for recalling and effecting at least one predetermined position of the central axis (4).

8. Medical apparatus according to claim 7, wherein displacing means are provided for the operating element (7) which upon actuation of the second control device (8) adjust the spatial orientation of the axis (9) of the operating element (7) according to the spatial orientation of the central axis (4).

9. Medical apparatus according to one of claims 1 to 8, wherein a VDU (10) is provided to indicate the spatial orientation of the central axis (4) and the spatial orientation of the axis (9) relative to a graphically represented subject to be examined.

10. Medical apparatus according to one of claims 1 to 9, wherein the operating element (7) can only assume those orientations which can also be assumed by the radiation transmitter (1).

## Revendications

1. Appareil médical comportant un émetteur de rayonnement (1), qui possède un axe central (4) et qui émet un rayon central le long de l'axe central (4), et dans lequel l'axe central (4) peut être orienté dans l'espace et, agit, au moyen d'un élément de commande (7) possédant un axe (9) pouvant être orienté dans l'espace, sur un dispositif de commande (6) pour modifier l'orientation spatiale de l'axe central (4), caractérisé par le fait que l'élément de commande (7) agit sur le dispositif de commande (6) de telle sorte que l'orientation spatiale de l'axe central (4) de l'émetteur de rayonnement (1) est en corrélation avec l'orientation spatiale de l'axe (9) de l'élément de commande.

2. Dispositif médical suivant la revendication 1, dans lequel l'élément de commande (7) est réalisé sous la forme d'une tige et l'orientation spatiale de l'axe central (4) est corrélée à l'orientation spatiale de l'axe longitudinal (9) de l'élément de commande (7).

3. Dispositif médical suivant la revendication 1 ou 2, qui comporte un récepteur de rayonnement (2) aligné sur l'émetteur de rayonnement (1), et dans lequel l'axe central (4) de l'émetteur de rayonnement (1) rencontre la zone centrale du récepteur de rayonnement (2).

4. Appareil médical suivant l'une des revendications 1 à 3, dans lequel un décalage de l'orientation spatiale de l'axe (9) de l'élément de commande (7) provoque un décalage de l'émetteur de rayonnement (1) de telle sorte que l'orientation spatiale de l'axe central (4) est asservie au décalage de l'axe (9) de l'élément de commande (7).

5. Dispositif médical suivant la revendication 4, dans lequel la vitesse de l'asservissement dépend de la différence entre l'orientation spatiale de l'axe (9) de l'élément de commande (7) et de l'orientation spatiale réelle de l'axe central (4), et dans lequel, dans le cas d'une différence importante, un asservissement est réalisé avec une vitesse élevée et, dans le cas d'une faible différence, un asservissement est réalisé avec une faible vitesse.

6. Dispositif médical suivant les revendications 4 et 5, dans lequel une orientation spatiale de l'axe central (4), qui correspond à l'orientation spatiale de l'axe (9) de l'élément de commande (7), est exécutée uniquement lors de l'actionnement d'un élément de commutation.

7. Dispositif médical suivant l'une des revendications 1 à 6, comportant un second dispositif de commande (8) pour appeler et exécuter au moins un alignement prédéterminé de l'axe central (4).

8. Dispositif médical suivant la revendication 7, dans lequel pour l'élément de commande (7) sont prévus des moyens de réglage, qui, lors d'une activation du second dispositif de commande (8), réalisent l'orientation spatiale de l'axe (9) de l'élément de commande (7) conformément à l'orientation spatiale de l'axe central (4).

9. Appareil médical suivant l'une des revendications 1 à 8, dans lequel il est prévu un dispositif d'affichage (10) pour l'orientation spatiale de l'axe central (4) ainsi que pour l'orientation spatiale de l'axe (9) par rapport à un objet d'examen représenté sous la forme d'une image.

10. Dispositif médical suivant l'une des revendications 1 à 9, dans lequel l'élément de commande (7) peut prendre uniquement des orientations qui peuvent être prises également par l'émetteur de rayonnement (1).
